# EUROPÄISCHE PATENTSCHRIFT

(11) **EP 0 506 651 B1**
(45) Veröffentlichungstag und Bekanntmachung des Hinweises auf die Patenterteilung: **16.10.1996**
(21) Anmeldenummer: 92890067.9
(22) Anmeldetag: 24.03.1992
(51) Int. Cl.: A61K 39/395, A61K 38/00

(54) **Pharmazeutische Präparation auf Basis von Plasmaproteinen**
Pharmaceutical preparation based on plasma proteins
Préparations pharmaceutiques sur la base de protéines plasmatiques

(30) Priorität: 25.03.1991 AT 651/91
(43) Veröffentlichungstag der Anmeldung: 30.09.1992
(73) Patentinhaber: IMMUNO Aktiengesellschaft, A-1221 Wien (AT)
(72) Erfinder: Eibl, Johann, Dr., A-1180 Wien (AT); Eibl, Martha, Prof. Dr., A-1180 Wien (AT); Linnau, Yendra, Dr., A-1224 Wien (AT)
(74) Vertreter: Wolfram, Gustav, Dipl.-Ing.

(56) Entgegenhaltungen:
- WO-A-90/06768
- GB-A- 1 499 078
- DATABASE WPIL Week 8829, Derwent Publications Ltd., London, GB; AN 88-198385
- DATABASE WPIL Week 8642, Derwent Publications Ltd., London, GB; AN 86-272127
- DATABASE WPIL Week 8641, Derwent Publications Ltd., London, GB; AN 86-265239

## Beschreibung

Die Erfindung betrifft eine pharmazeutische Präparation mit antimikrobieller, antiphlogistischer bzw. antiallergischer Wirkung auf Basis von Plasmaproteinen.

Pharmazeutische Präparationen auf Basis von Immunglobulinen werden bereits zur Prophylaxe und Behandlung bakterieller und viraler Infektionen verwendet. In der US-A- 4 335 099 beispielsweise wird ein oral verabreichbares Präparat beschrieben, das Immunglobulin A (IgA) und Immunglobulin G (IgG) enthält und das sich zur Behandlung von Darminfektionen eignet. Es ist weiters bekannt, daß eine Immunglobulin-Präparation mit einem IgG- und IgA-Anteil am Gesamtglobulin von 73 bzw. von 26 % bei prophylaktischer Verabreichung an Frühgeborene die Gefahr einer Erkrankung an nekrotisierender Enterocolitis mindern kann (Martha M. Eibl et al., New Journal of Medicine, 319, S. 1 - 7, 1988).

Es wurde auch nachgewiesen, daß Serum eine bakteriostatische Wirkung auf Staphylococcus aureus aufweist, wobei diese Wirkung von einer Sättigung des im Serum enthaltenen Siderophilins (Transferrins) mit Eisen abhängt (Arthur L. Schade, Protides Biolog. Fluids; Proc. Colloq. 1961, 8, S. 261 - 263). Diese mit Eisen angereicherte Serum- Probe enthält pro Gewichtsteil IgA 15 Gewichtsteile IgG und 2,6 Gewichtsteile Transferrin und wirkt nur gegen wenige Erreger.

Auch alle bekannten IgA - hältigen pharmazeutischen Präparationen haben ein relativ schmales Wirkungsspektrum.

Die Erfindung setzt sich zum Ziel, eine pharmazeutische Präparation mit antimikrobieller Wirkung auf Basis von Plasmaproteinen zur Verfügung zu stellen, die ein breites Wirkungsspektrum aufweist. Außerdem soll diese Präparation auch eine antiphlogistische und antiallergische Wirkung besitzen.

Die erfindungsgemäße pharmazeutische Präparation ist dadurch gekennzeichnet, daß sie Immunglobulin A (IgA), Immunglobulin G (IgG) und Transferrin enthält, wobei pro Gewichtsteil IgA zwischen 0,40 und 0,80 Gewichtsteile IgG und zwischen 0,15 und 0,45 Gewichtsteile Transferrin vorgesehen sind.

Es hat sich gezeigt, daß sowohl die Stärke der Wirkung auf bakterielle und virale Erreger, als auch das Wirkungsspektrum einer IgA- und IgG - hältigen Präparation vergrößert werden kann, wenn sie noch zusätzlich Transferrin enthält und die drei Komponenten IgA, IgG und Transferrin in den erfindungsgemäßen Gewichtsverhältnissen vorliegen. Diese Verhältnisse sind jenen in der Muttermilch und im Kolostrum vergleichbar.

In der erfindungsgemäßen Präparation liegt das IgA bevorzugt in einer Form vor, in der es das sogenannte "Secretory Component" enthält, ein Protein, das dem IgA die Fähigkeit verleiht, Schleimhaut zu durchdringen.

Eine bevorzugte Ausführungsform der erfindungsgemäßen Präparation ist dadurch gekennzeichnet, daß sie pro Milliliter applikationsbereiter Lösung zwischen 10 und 100 mg IgA, zwischen 4 und 80 mg IgG und zwischen 1,5 und 45 mg Transferrin enthält.

Eine weitere bevorzugte Ausführungsform der erfindungsgemäßen pharmazeutischen Präparation ist durch eine oder mehrere Produktionsstufen erhältlich, in welchen infektiöse Agentien, insbesondere Viren, inaktiviert bzw. entfernt wurden.

Die erfindungsgemäße Präparation eignet sich insbesondere zur Prophylaxe und Therapie von Schleimhautinfektionen, insbesondere von nekrotisierender Enterocolitis, von Entzündungen und Allergien. Sie kann außerdem Muttermilch in ihrer Funktion bei der Vermittlung von humoraler Immunität an Säuglinge ersetzen.

Zur Herstellung der erfindungsgemäßen Präparation können handelsübliches IgA und IgG, handelsübliche IgA/IgG-Mischpräparate und handelsübliches Transferrin verwendet werden.

Ein bevorzugtes Herstellungsverfahren ist dadurch gekennzeichnet, daß
- eine IgA, IgG und Transferrin enthaltende Plasmafraktion zur Inaktivierung von infektiösen Agentien behandelt oder in unbehandelter Form mit einem Ionenaustauscher in Kontakt gebracht wird, um IgA, IgG und Transferrin zu adsorbieren,
- das am Ionenaustauscher adsorbierte IgA, IgG und Transferrin gewaschen und eluiert wird,
- das Eluat einer Reinigungsfällung zur Abtrennung unerwünschter Begleitproteine unterworfen wird, um eine gereinigte IgA-, IgG- und Transferrin-hältige Lösung zu erhalten,
- die erhaltene Lösung mit einem Proteinfällungsmittel behandelt wird, um IgA, IgG und Transferrin zu fällen und um eine Vorinaktivierung eventuell vorhandener infektiöser Agentien zu bewirken,
- das gefällte IgA, IgG und Transferrin abgetrennt, gefriergetrocknet, zur weiteren Inaktivierung infektiöser Agentien behandelt und schließlich in an sich bekannter Weise zu einer oral, parenteral oder topisch anwendbaren pharmazeutischen Präparation aufgearbeitet wird.

Es hat sich gezeigt, daß es während der genannten Verfahrensschritte zu einer Reduktion eines im Ausgangsmaterial eventuell vorhandenen Titers an infektiösen Agentien, wie Viren, kommt. Dieser Effekt ist dann besonders ausgeprägt, wenn die erfindungsgemäße Präparation hergestellt wird, indem
- Plasma oder Plasmafraktionen nach der Methode von Cohn mit Äthanol behandelt werden, um die Cohn II+III - Fraktion abzutrennen,
- die abgetrennte Cohn II+III - Fraktion mit einem wässerigen Puffer bei einem pH zwischen 5,0 und 6,5 digeriert wird, um ein Proteingemisch enthaltend IgA, IgG und Transferrin zu extrahieren,
- das extrahierte Proteingemisch mit Äthanol versetzt wird, um unerwünschte Begleitproteine zu fällen,
- die gefällten Begleitproteine abgetrennt werden, um eine IgA-, IgG- und Transferrin enthaltende Lösung zu erhalten,
- diese IgA, IgG und Transferrin enthaltende Lösung mit einem DEAE - Ionenaustauscher in Kontakt gebracht wird, um IgA, IgG und Transferrin zu adsorbieren,
- das adsorbierte IgA, IgG und Transferrin gewaschen und eluiert wird,
- das erhaltene Eluat mit Äthanol behandelt wird, um IgA und IgG zu fällen,
- das gefällte IgA und IgG abgetrennt wird, um einen IgA-und IgG-hältigen Niederschlag und einen Transferrin-hältigen Überstand zu erhalten,
- der Überstand angesäuert wird, um Transferrin zu fällen,
- das gefällte Transferrin abgetrennt wird,
- die abgetrennten Niederschläge vereinigt und zu einer pharmazeutischen Präparation fertiggestellt werden.

Diese Variante des erfindungsgemäßen Verfahrens führt bei eventueller Anwesenheit von infektiösen Agentien im Ausgangsmaterial zu einer Reduktion des Titers von mindestens 29 Logarithmuseinheiten.

Nachstehend werden die Herstellung der erfindungsgemäßen Präparation und ihre Wirksamkeit näher beschrieben.

### Herstellung

### Beispiel 1

100 1 gefrorenes Humanplasma werden aufgetaut, der Niederschlag (Kryopräzipitat) abgetrennt und der Überstand (Kryoüberstand) bei -2°C und einem pH-Wert von 7,0 mit Ethanol bis zu einer Konzentration von 8 % versetzt. Nach Abtrennung des dabei entstehenden Niederschlages wird die Ethanol-Konzentration des Überstandes bei -5°C auf 25 % erhöht. Der dabei ausfallende Niederschlag (Cohn II+III-Fraktion) wird mit einem Phosphat-Acetat-Puffer extrahiert und mit 12 % Ethanol bei einem pH-Wert von 5,3 und einer Temperatur von -2°C versetzt, wobei sich ein Niederschlag bildet, der abgetrennt wird. Der Überstand wird bei einem pH-Wert zwischen 6,0 und 6,5 mit einem Anionenaustauscher wie DEAE - Sephadex® behandelt, um IgA, teilweise IgG und Transferrin zu adsorbieren. Adsorbiert werden auch noch andere Proteine wie Albumin.

Zur Entfernung der nicht gebundenen Proteine wird gewaschen, wobei über die Intensität des Waschschrittes das Verhältnis von IgA zu IgG im Endprodukt beeinflußt werden kann. Danach werden die adsorbierten Proteine mit einer phosphatgepufferten, 3 %-igen NaCl-Lösung eluiert. Die erhaltene Lösung wird bei einem pH-Wert zwischen 6,4 und 6,5 und einer Temperatur von -3°C mit 20 % Ethanol versetzt, der sich dabei bildende Niederschlag verworfen und im Überstand die Ethanolkonzentration auf 30 % erhöht, wobei IgA, IgG und Transferrin ausfallen.

Dieser Niederschlag wird abgetrennt, in Wasser gelöst, und bei einem pH-Wert von 6,5 und einer Temperatur von -5°C mit 25 % Ethanol versetzt. Das sich dabei bildende Präzipitat enthält IgA und IgG und wird abgetrennt. Transferrin bleibt in Lösung und wird durch Einstellen des pH-Wertes auf 5,5 gefällt.

Dann werden in den Niederschlägen nach bekannten Methoden die Gehalte an IgA, IgG und Transferrin bestimmt und durch Zusammenmischen der entsprechenden Mengen die erfindungsgemäße Zusammensetzung eingestellt, wobei gegebenenfalls IgA, IgG und/oder Transferrin mit handelsüblichen Präparaten ergänzt und noch pharmazeutische Zusatzstoffe zugegeben werden können.

Anschließend wird die Lösung gefriergetrocknet, mit Wasser befeuchtet und 10 Stunden bei 60°C hitzebehandelt, um eventuell vorhandene Krankheitserreger abzutöten.

Um das gefriergetrocknete, hitzebehandelte Pulver in eine applizierbare Form zu bringen, kann es entweder in einer NaCl-hältigen Glukoselösung gelöst, sterilfiltriert und abgefüllt werden, oder es kann zur oralen Verabreichung tablettiert oder in Kapseln gefüllt werden, wobei jedoch Tabletten und Kapseln mit einer magensaftresistenten Schicht überzogen werden müssen.

### Beispiel 2

10 l Humanplasma werden mit 140 g Ammoniumsulfat pro Liter bei pH 7,2 versetzt. Nach Abtrennung des Niederschlages wird die Ammoniumsulfatkonzentration auf 275 g/l erhöht. Der dabei ausfallende Niederschlag wird in einem 5 mMol/l Azetatpuffer bei pH 5,0 gelöst, der unlösliche Anteil abgetrennt und verworfen. Aus dem Überstand wurden IgA, IgG und Transferrin isoliert und durch Ausfällung mit 30 % Äthanol bei pH 6,6 und -5 °C konzentriert.

Das Präparat besitzt folgende Zusammensetzung:

| | |
|---|---|
| IgA | 63 mg/ml |
| IgG | 28 mg/ml |
| Transferrin | 10 mg/ml |

### Reduktion des Virus-Titers

Zur Dokumentation der Inaktivierung bzw. der Entfernung eventuell im Ausgangsplasma vorhandener Viren während der Herstellung der erfindungsgemäßen Präparation, wurden die im Beispiel 1 beschriebenen Verfahrensschritte wiederholt, wobei vor jedem einzelnen Verfahrensschritt in der Probe ein bestimmter Virus-Titer (HIV) eingestellt und nach Durchführung der Verfahrensschritte bestimmt wurde. Die Ergebnisse sind in der Tabelle 1 zusammengefaßt.

### Schritt 1: Gewinnung des Kryopräzipitats

10 ml humanes Plasma wurden mit 1 ml einer HIV-1-Virussuspension, enthaltend 10⁶ Infektionseinheiten pro 0,5 ml (Virussuspension I) versetzt. Die Mischung wurde bei -20 °C eingefroren und zwischen 0 bis 2 °C aufgetaut. Der gebildete Niederschlag wurde durch Zentrifugation abgetrennt. Im Überstand (Kryoüberstand) wurde der Virustiter mit 10⁴ Infektion Einheiten/ml bestimmt. Im Verfahrensschritt 1 wird somit eine Reduktion des Virus-Titers um eine Zehnerpotenz erreicht.

### Schritt 2: Fraktionierung mit 8 % Äthanol

6 ml Kryoüberstand wurden mit 0,6 ml Virus Suspension I versetzt. Die Temperatur der Mischung wurde bei -1 bis -2 °C gehalten und 0,6 ml eines 96 %igen Äthanols (-20 °C) zugesetzt, sodaß eine 8 %ige Äthanolkonzentration vorliegt. Der entstandene Niederschlag wurde durch Zentrifugation abgetrennt. Im Überstand wurde die Viruskonzentration mit 10³ Infektion Einheiten/ml bestimmt, was eine Reduktion um den Faktor 10² bedeutet.

### Schritt 3: Trennung des Albumins von den Immunglobulinen

8 ml des Überstandes von Schritt 2 wurden mit 0,8 ml einer HIV-1-Virussuspension, enthaltend 10⁷ Infektionseinheiten pro 0,5 ml (Virussuspension II) versetzt. Die Temperatur der Mischung wurde bei -5 °C gehalten und 2,1 ml einer 96 %igen Äthanollösung zugesetzt um eine 25 %ige Lösung zu bekommen. Der gebildete Niederschlag wurde durch Zentrifugation abgetrennt. Im gelösten Niederschlag (in 1 ml PBS) konnte kein Virus nachgewiesen werden, was einer Reduktion um den Faktor 10⁶ bedeutet.

### Schritt 4: Extraktion und Fällung mit 12 % Ethanol

1 g des im Schritt 3 erhaltenen Niederschlags wurde in 30 ml Phosphat-Acetat-Puffer suspendiert. 10 ml dieser Suspension wurde mit 1 ml der Virussuspension I versetzt. Bei einer Temperatur von -2°C wurden 1,6 ml einer 96 %igen Ethanollösung zugesetzt, wobei die Endkonzentration 12 % Masse betrug. Diese Suspension wurde zentrifugiert. Im Überstand konnte kein Virus nachgewiesen werden, was einer Reduktion um den Faktor 10⁵ entspricht.

### Schritt 5: Adsorption, Waschung, Eluation

Zu 10 ml des Überstandes vor Schritt 4 wurden 500 mg DEAE-Sephadex® pro Gramm Protein zugesetzt, mit 1 ml der Virussuspension II versetzt, bei -2°C gerührt und das Adsorbat abgetrennt; das Sephadex® wurde gewaschen und die Proteine eluiert.

Der Virus Titer betrug 10³ Infektionseinheiten pro 3 ml, was einer Reduktion um den Faktor 10³ entspricht.

### Schritt 6: Fällung mit 20 % Ethanol

5 ml des Eluates aus Schritt 5 wurden mit 0,5 ml Virussuspension II versetzt, 1,45 ml einer 96 %igen Ethanollösung zugesetzt, bei -2°C gerührt und der Niederschlag abzentrifugiert. Im Überstand konnte kein Virus nachgewiesen werden (Reduktion: 10⁶).

### Schritt 7: Fällung mit 30 % Ethanol

5 ml Überstand aus Schritt 6 wurden mit 0,5 ml Virussuspension II und 0,83 ml Ethanol versetzt, bei -5°C gerührt und zentrifugiert. Der Niederschlag wurde in 1 ml Puffer gelöst. Es konnte kein Virus nachgewiesen werden (Reduktion: 10⁶).

Die gesamte Virustiter-Reduktion (Summe der Verfahrensschritte 1 bis 7) betrug somit mindestens 10²⁹ (siehe Tabelle).

**Tabelle 1**

| Reduktion des Virustiters | | | |
|---|---|---|---|
| Verfahrensschritt | Virus Titer Inf.Einh/ml | | Reduktion des Virus-Titer |
| | vor | nach | |
| | Verfahrensschritt | | |
| 1 | 10⁵ | 10⁴ | 10¹ |
| 2 | 10⁵ | 10³ | 10² |
| 3 | 10⁶ | 0 | 10⁶ |
| 4 | 10⁵ | 0 | > 10⁵ |
| 5 | 10⁶ | 10³ | 10³ |
| 6 | 10⁶ | 0 | > 10⁶ |
| 7 | 10⁶ | 0 | > 10⁶ |
| | Gesamte Virustiter-Reduktion | | > 29 |

### Wirksamkeit gegen Bakterien

Zur Prüfung auf Wirksamkeit der erfindungsgemäßen Präparation auf Bakterien wurden drei Lösungen (a, b und c) der in Tab. 2 angegebenen Zusammensetzung verwendet:

**Tabelle 2**

| Lösung | IgA | IgG | Transferrin |
|---|---|---|---|
| a | 50 | 30 | 15 |
| b | 25 | 20 | 11 |
| c | 90 | 40 | 15 |
| (Zahlenangaben in mg/ml) | | | |

3 ml der jeweiligen Lösung a, b und c wurden in Röhrchen mit je 0,1 ml verschiedener Bakteriensuspensionen Staphylococcus aureus, Escherichia coli, Pseudomonas aeruginosa, Bacillus subtilis sowie einer Suspension des pathogenen Hefepilzes Candida albicans geimpft, geschüttelt, 2 x 0,1 ml in Petrischalen pipettiert und mit Nutrientagar bzw. für Candida mit Sojabohnen-Caseinagar ausgegossen.

Nach einer Inkubationszeit von 0,5; 1; 3; 5 und 24 Stunden bei 20 bis 25°C wurden die Kolonien ausgezählt. Alle drei Präparationen a, b und c hemmten das Wachstum aller fünf Mikroorganismen. Nur bei Lösung b war die Wachstumshemmung auf E.Coli schwach ausgeprägt.

Zum Vergleich wurden die Wirkungen von Albumin, drei IgA/IgG-Mischungen und von Transferrin getestet. Die Ergebnisse sind in der Tabelle 3 angeführt.

**Tabelle 3**

| | Staph. aur. | E.coli | Pseud. aerug. | Bac.sub. | cand.alb. |
|---|---|---|---|---|---|
| Albumin (50mg); | - | - | - | - | - |
| IgA (56mg) IgG (2mg); | - | + | - | - | - |
| IgA (75mg) IgG (26mg); | + | - | - | - | + |
| IgA (3mg) IgG (100mg); | - | - | - | + | - |
| Transferrin (56mg); | - | - | + | - | - |
| Transferrin (15mg); | - | - | - | - | - |
| Die Zahlen beziehen sich auf mg pro Milliliter Lösung -: keine Hemmung Wachstums der Mikroorganismen +: Hemmung des Wachstums der Mikroorganismen | | | | | |

### Wirksamkeit gegen Viren

Zur Feststellung der Wirkung der erfindungsgemäßen Präparation auf Viren wurden je 1 ml der oben bechriebenen Lösung a mit 0,1 ml HIV-, Sindbis- und VSV-Virussuspension versetzt, gefriergetrocknet, befeuchtet und 10 Stunden bei 60°C in verschlossenen Behältnissen erhitzt, wobei vor und nach der Hitzebehandlung der Virustiter bestimmt wurde. Die Ergebnisse sind in Tab. 4 zusammengefaßt.

**Tabelle 4**

| Virus | Virus-Titer vor Beginn der Behandlung | nach 10 Stunden |
|---|---|---|
| Sindbis | 10⁵ | < 10^{0,5} |
| VSV | 10^{4,4} | < 10^{o,5} |
| HIV | 10⁴ | < 10^{0,5} |

## Patentansprüche

1. Pharmazeutische Präparation mit antimikrobieller, antiphlogistischer bzw. antiallergischer Wirkung auf Basis von Plasmaproteinen, dadurch gekennzeichnet, daß die Präparation Immunglobulin A (IgA), Immunglobulin G (IgG) und Transferrin enthält, wobei pro Gewichtsteil IgA zwischen 0,40 und 0,80 Gewichtsteile IgG und zwischen 0,15 und 0,45 Gewichtsteile Transferrin vorgesehen sind.

2. Pharmazeutische Präparation nach Anspruch 01, dadurch gekennzeichnet, daß das IgA das "Secretory Component" enthält.

3. Pharmazeutische Präparation nach Anspruch 1, dadurch gekennzeichnet, daß sie pro Milliliter applikationsfertiger Lösung zwischen 10 und 100 mg, vorzugsweise zwischen 45 und 55 mg, IgA, zwischen 4 und 80 mg, vorzugsweise zwischen 25 und 35mg, IgG und zwischen 1,5 und 45 mg, vorzugsweise zwischen 10 und 20 mg, Transferrin enthält.

4. Pharmazeutische Präparation nach einem oder mehreren der Ansprüche 1 bis 3, erhältlich durch eine oder mehrere Produktionsstufen, in welchen infektiöse Agentien, insbesondere Viren, inaktiviert bzw. entfernt wurden.

5. Pharmazeutische Präparation nach einem oder mehreren der Ansprüche 1 bis 4 zur Prophylaxe und Therapie von Schleimhautinfektionen, insbesondere von nekrotisierender Enterocolitis, von Entzündungen und Allergien.

6. Verfahren zur Herstellung einer pharmazeutischen Präparation nach Anspruch 1, gekennzeichnet durch die Kombination der Maßnahmen, daß
- eine IgA, IgG und Transferrin enthaltende Plasmafraktion zur Inaktivierung von infektiösen Agentien behandelt oder in unbehandelter Form mit einem Ionenaustauscher in Kontakt gebracht wird, um IgA, IgG und Transferrin zu adsorbieren,
- das am Ionenaustauscher adsorbierte IgA, IgG und Transferrin gewaschen und eluiert wird,
- das Eluat einer Reinigungsfällung zur Abtrennung unerwünschter Begleitproteine unterworfen wird, um eine gereinigte IgA-, IgG- und Transferrin-hältige Lösung zu erhalten,
- die erhaltene Lösung mit einem Proteinfällungsmittel behandelt wird, um IgA, IgG und Transferrin zu fällen und um eine Vorinaktivierung eventuell vorhandener infektiöser Agentien zu bewirken,
- das gefällte IgA, IgG und Transferrin abgetrennt, gefriergetrocknet, zur weiteren Inaktivierung infektiöser Agentien behandelt und schließlich in an sich bekannter Weise zu einer oral, parenteral oder topisch anwendbaren pharmazeutischen Präparation aufgearbeitet wird.

7. Verfahren nach Anspruch 6, dadurch gekennzeichnet, daß
- Plasma oder Plasmafraktionen nach der Methode von Cohn mit Äthanol behandelt werden, um die Cohn II+III - Fraktion abzutrennen,
- die abgetrennte Cohn II+III - Fraktion mit einem wässerigen Puffer bei einem pH zwischen 5,0 und 6,5 digeriert wird, um ein Proteingemisch enthaltend IgA, IgG und Transferrin zu extrahieren,
- das extrahierte Proteingemisch mit Äthanol versetzt wird, um unerwünschte Begleitproteine zu fällen,
- die gefällten Begleitproteine abgetrennt werden, um eine IgA-, IgG- und Transferrin enthaltende Lösung zu erhalten,
- diese IgA, IgG und Transferrin enthaltende Lösung mit einem DEAE - Ionenaustauscher in Kontakt gebracht wird, um IgA, IgG und Transferrin zu adsorbieren,
- das adsorbierte IgA, IgG und Transferrin gewaschen und eluiert wird,
- das erhaltene Eluat mit Äthanol behandelt wird, um IgA und IgG zu fällen,
- das gefällte IgA und IgG abgetrennt wird, um einen IgA- und IgG-hältigen niederschlag und einen Transferrin-hältigen Überstand zu erhalten,
- der Überstand angesäuert wird, um Transferrin zu fällen,
- das gefällte Transferrin abgetrennt wird,
- die abgetrennten Niederschläge vereinigt und zu einer pharmazeutischen Präparation fertiggestellt werden.

## Claims

1. A pharmaceutical preparation based on plasmaproteins and having an antimicrobial, antiphlogistic or antiallergic activity, respectively, characterised in that the preparation contains immunoglobulin A (IgA), immunoglobulin G (IgG) and transferrin, wherein between 0.40 and 0.80 parts by weight of IgG and between 0.15 and 0.45 parts by weight of transferrin are provided per part by weight of IgA.

2. A pharmaceutical preparation according to claim 1, characterised in that the IgA contains the "secretory component".

3. A pharmaceutical preparation according to claim 1, characterised in that it contains between 10 and 100 mg, preferably between 45 and 55 mg, of IgA, between 4 and 80 mg, preferably between 25 and 35 mg, of IgG, and between 1.5 and 45 mg, preferably between 10 and 20 mg, of transferrin per milliliter of solution ready for application.

4. A pharmaceutical preparation according to one or several of claims 1 to 3 obtainable by one or several production stages, in which infectious agents, in particular viruses, have been inactivated or removed, respectively.

5. A pharmaceutical preparation according to one or several of claims 1 to 4 for the prevention and therapy of mucous membrane infections, in particular necrotizing enterocolitis, inflammations and allergies.

6. A method of producing a pharmaceutical preparation according to claim 1, characterised by the combination of the measures that
- a plasma fraction containing IgA, IgG and transferrin, which has been treated for the inactivation of infectious agents, or in the untreated form, is contacted with an ion exchanger so as to adsorb IgA, IgG and transferrin,
- the IgA, IgG and transferrin adsorbed on the ion exchanger are washed and eluted,
- the eluate is subjected to a purifying precipitation for separating undesired accompanying proteins so as to obtain a purified IgA, IgG and transferrin containing solution,
- the solution obtained is treated with a protein precipitating agent to precipitate IgA, IgG and transferrin and to effect a pre-inactivation of possibly present infectious agents,
- the precipitated IgA, IgG and transferrin are separated, freeze-dried, treated for further inactivation of infectious agents, and finally are processed in a manner known per se to an orally, parenterally or topically applicable pharmaceutical preparation.

7. A method according to claim 6, characterised in that
- plasma or plasma fractions are treated with ethanol according to the method of Cohn so as to separate the Cohn II+III fraction,
- the separated Cohn II+III fraction is treated with an aqueous buffer at a pH of between 5.0 and 6.5 so as to extract a protein mixture containing IgA, IgG and transferrin,
- the extracted protein mixture is mixed with ethanol so as to precipitate undesired accompanying proteins,
- the precipitated accompanying proteins are separated so as to obtain a solution containing IgA, IgG and transferrrin,
- this IgA, IgG and transferrin containing solution is contacted with a DEAE ion exchanger so as to adsorb IgA, IgG and transferrin,
- the adsorbed IgA, IgG and transferrin are washed and eluted,
- the eluate obtained is treated with ethanol so as to precipitate IgA and IgG,
- the precipitated IgA and IgG are separated so as to obtain an IgA- and IgG-containing precipitate and a transferrin-containing supernatant,
- the supernatant is acidified so as to precipitate transferrin,
- the precipitated transferrin is separated,
- the separated precipitates are combined and completed to a pharmaceutical preparation.

## Revendications

1. Préparation pharmaceutique à effet antimicrobien, antiphlogistique et antiallergique à base de protéines plasmatiques, caractérisée en ce que la préparation contient des immunoglobulines A (IgA), des immunoglobulines G (IgG) et de la transferrine, entre 0,40 et 0,80 partie en masse d'IgG et entre 0,15 et 0,45 partie en masse de transferrine étant prévues par partie en masse d'IgA.

2. Préparation pharmaceutique selon la revendication 1, caractérisée en ce que les IgA contiennent le "composant sécrétoire".

3. Préparation pharmaceutique selon la revendication 1, caractérisée en ce qu'elle contient par millilitre de solution prête à l'application entre 10 et 100 mg, de préférence entre 45 et 55 mg, d'IgA, entre 4 et 80 mg, de préférence entre 25 et 35 mg, d'IgG et entre 1,5 et 45 mg, de préférence entre 10 et 20 mg, de transferrine.

4. Préparation pharmaceutique selon une ou plusieurs des revendications 1 à 3, qui peut être obtenue par une ou plusieurs étapes de production dans lesquelles les agents infectieux, en particulier les virus, ont été inactivés ou éliminés.

5. Préparation pharmaceutique selon une ou plusieurs des revendications 1 à 4 pour la prophylaxie et la thérapie des infections des muqueuses, en particulier de l'entérocolite nécrosante, des inflammations et des allergies.

6. Procédé d'obtention d'une préparation pharmaceutique selon la revendication 1, caractérisé par la combinaison des dispositions selon lesquelles
- une fraction plasmatique contenant des IgA, des IgG et de la transferrine pour l'inactivation des agents infectieux sous forme traitée ou non traitée est mise en contact avec un échangeur d'ions pour adsorber les IgA, les IgG et la transferrine,
- les IgA, les IgG et la transferrine adsorbées sur l'échangeur d'ions sont lavées et éluées,
- l'éluat est soumis à une précipitation de purification pour la séparation des protéines d'accompagnement indésirables, pour obtenir une solution contenant des IgA, des IgG et de la transferrine purifiées,
- la solution obtenue est traitée avec un agent de précipitation des protéines pour précipiter les IgA, les IgG et la transferrine et pour réaliser une pré-inactivation des agents infectieux éventuellement présents,
- les IgA, les IgG et la transferrine précipitées sont séparées, lyophilisées, traitées pour l'inactivation supplémentaire des agents infectieux et enfin mises d'une manière connue en soi sous forme d'une préparation pharmaceutique qui peut être utilisée par voie orale, parentérale ou topique.

7. Procédé selon la revendication 6, caractérisé en ce que
- du plasma est traité à l'éthanol, ou des fractions plasmatiques sont traitées à l'éthanol, selon la méthode de Cohn pour séparer la fraction de Cohn II+III,
- la fraction de Cohn II+III séparée est digérée avec un tampon aqueux à un pH compris entre 5,0 et 6,5 pour extraire un mélange de protéines contenant les IgA, les IgG et la transferrine,
- le mélange de protéines extrait est additionné d'éthanol pour précipiter les protéines d'accompagnement indésirables,
- les protéines d'accompagnement précipitées sont séparées pour obtenir une solution contenant les IgA, les IgG et la transferrine,
- cette solution contenant les IgA, les IgG et la transferrine est mise en contact avec un échangeur d'ions DEAE pour adsorber les IgA, les IgG et la transferrine,
- les IgA, les IgG et la transferrine adsorbées sont lavées et éluées,
- l'éluat obtenu est traité à l'éthanol pour précipiter les IgA et les IgG,
- les IgA et les IgG précipitées sont séparées pour obtenir un précipité contenant les IgA et les IgG et un sumageant contenant la transferrine,
- le surnageant est acidifié pour précipiter la transferrine,
- la transferrine précipitée est séparée,
- les précipités séparés sont réunis et mis sous la forme finale d'une préparation pharmaceutique.
